# EUROPEAN PATENT APPLICATION

(11) **EP 4 115 914 A1**
(43) Date of publication of application: **11.01.2023**
(21) Application number: 21765054.8
(22) Date of filing: 05.03.2021
(51) Int. Cl.: A61L 27/10, A61L 27/12, A61L 27/56, A61F 2/28

(54) **MEDICAL HONEYCOMB STRUCTURE AND MANUFACTURING METHOD THEREOF, MEDICAL TISSUE RECONSTRUCTION BAG, AND FORMING MOLD**

(30) Priority: 05.03.2020 JP 2020038167
(71) Applicant: GC Corporation, Sunto-gun, Shizuoka 410-1307 (JP)
(72) Inventor: HAYASHI Koichiro, Fukuoka-shi, Fukuoka 812-0064 (JP); TSUCHIYA Akira, Fukuoka-shi, Fukuoka 812-0044 (JP); KISHIDA Ryo, Fukuoka-shi, Fukuoka 812-0051 (JP); NAKASHIMA Yasuharu, Fukuoka-shi, Fukuoka 813-0017 (JP); ISHIKAWA Kunio, Kasuya-gun, Fukuoka 811-2201 (JP)
(74) Representative: TBK
(86) International application number: PCT/JP2021/008783
(87) International publication number: WO 2021/177457

(57) **Abstract**

It is to provide a medical honeycomb structure lacking at least a portion of an outer peripheral side wall of a honeycomb structure comprising
a plurality of through-holes extending in one direction, wherein sites lacking the outer peripheral side wall comprise a plurality of grooves, and comprise a plurality of planes including distant surfaces of groove side walls flanked by the grooves.

## Description

### Technical Field

The present invention relates to a medical honeycomb structure for use in tissue reconstruction surgery for bones and the like, or in scaffolds and the like for regenerative medicine, in the medical field or fields related to medicine, and a method for producing the same, a medical tissue reconstruction bag, and a molding die.

### Background Art

In clinical practice, tissue defects may be reconstructed with medical materials. Although required properties differ depending on medical materials, medical tissue reconstruction materials used in the reconstruction of tissue defects may be desired to satisfy most of the following demands (1) to (7):
(1) the medical tissue reconstruction materials can easily reconstruct a tissue defect and are less likely to move away from the tissue defect;
(2) the materials strongly bind to surrounding tissues when implanted *in vivo;*
(3) the materials elicit large defected tissue reconstruction rate in the tissue defect;
(4) the materials exhibit an ability to induce orientation of the surrounding tissues;
(5) the materials have large mechanical strength;
(6) the materials are inexpensive; and
(7) the materials are replaced with tissues.

The demand (1) relates to an operative procedure, and deviation immediately after the surgery. Medical tissue reconstruction materials that enable easy procedures of tissue reconstruction surgery are highly useful. On the other hand, medical tissue reconstruction materials result in poor prognosis if they move from a tissue defect after the surgery.

For tissue reconstruction surgery, the level of the demand (2) is high. If the medical tissue reconstruction materials cannot rapidly bind to surrounding tissues after the surgery, the medical tissue reconstruction materials move away from implantation sites, resulting in poor prognosis.

The demand (3) relates to the defected tissue reconstruction rate. For example, in bone reconstruction surgery, a bone defect may be reconstructed with connective tissues, not bone tissues. However, a higher ratio at which such a bone defect is reconstructed with bone tissues is more preferred.

The demand (4) is for reconstruction surgery of oriented tissues such as bones, nerves, and the like. The tissues of bones, nerves, and the like are variously oriented depending on sites, and such orientation of tissues improves functions (Non Patent Literatures 1 to 3).

The demands (5) and (6) are common in medical materials.

The demand (7) relates to a higher level of tissue regeneration techniques in tissue reconstruction surgery. The absorption of medical tissue reconstruction materials and their replacement with tissues based on new tissue formation depend basically on material composition, whereas the replacement rates of materials to be replaced with tissues depend on the structures of the materials.

Although medical materials that satisfy all the demands (1) to (7) are ideal, medical materials that satisfy most of these demands or medical tissue reconstruction materials that partially satisfy these demands at a high level are also useful.

Patent Literatures 1 to 3 have each proposed a medical material comprising grooves in the same direction. However, the medical material is not designed such that surrounding tissues can penetrate into the material. Thus, a strong binding between a plane perpendicular to the groove direction and surrounding tissues cannot be expected. Therefore, the demands are satisfied only at a limited level.

Patent Literatures 4 to 6 have each proposed a method for producing an orientational interconnected porous material by exploiting the principle of ice columns. However, the orientational interconnected porous material produced by exploiting the principle of ice columns has small mechanical strength because walls are not continuous. Furthermore, strict temperature control is necessary for the growth of ice columns, resulting in poor productivity and a high production cost. Such a material is not classified as a honeycomb structure.

For example, Patent Literatures 7 and 8 have each proposed a honeycomb structure comprising a plurality of through-holes extending in one direction. However, the honeycomb structure has an outer peripheral side wall. Therefore, a high binding force between the outer peripheral side wall and surrounding tissues cannot be expected.

Patent Literature 9 has proposed a honeycomb structure comprising a plurality of through-holes that penetrate an outer peripheral side wall. Patent Literature 10 has proposed a honeycomb structure having a plurality of grooves on the outer peripheral section produced by cutting the honeycomb structure at one plane parallel to the through-hole direction, and removing an outer peripheral side wall. Patent Literature 11 has proposed a honeycomb structure, wherein an outer peripheral section has grooves formed by lacking side walls of through-holes, and through-hole inlets adjacent to the grooves. However, a problem of the production of these honeycomb structures is a high production cost because an additional step of conferring the structure is essential.

### Citation List

### Patent Literatures

Patent Literature 1: Japanese Patent Laid-Open Publication No. 63 (1988) -119757
Patent Literature 2: Japanese Patent Laid-Open Publication No. 01 (1989) -166765
Patent Literature 3: Japanese Patent Laid-Open Publication No. 2002-519074
Patent Literature 4: Japanese Patent Laid-Open Publication No. 2008-230910
Patent Literature 5: Japanese Patent Laid-Open Publication No. 2010-018459
Patent Literature 6: Japanese Patent Laid-Open Publication No. 2012-148929
Patent Literature 7: Japanese Patent Laid-Open Publication No. 2004-298407
Patent Literature 8: Japanese Patent Laid-Open Publication No. 2005-152006
Patent Literature 9: Japanese Patent Laid-Open Publication No. 2005-110709
Patent Literature 10: Japanese Patent Laid-Open Publication No. 2004-298545
Patent Literature 11: International Publication No. WO 2018/074429

### Non-Patent Literatures

Non-Patent Literature 1: Nakano T. et al, "Unique alignment and texture of biological apatite crystallites in typical calcified tissues analyzed by micro-beam X-ray diffractometer system" Bone, 31[4] (2002) 479-487.
Non-Patent Literature 2: Nakano T. et al, "Biological apatite (BAp) crystallographic orientation and texture as a new index for assessing the microstructure and function of bone regenerated by tissue engineering" Bone 51 (2012) 741-747.
Non-Patent Literature 3: Ishimoto T. et al "Degree of biological apatite c-axis orientation rather than bone mineral density controls mechanical function in bone regenerated using rBMP-2" Journal of Bone and Mineral Research 28 (2013) 1170-1179.

### Summary of Invention

### Technical Problem

As mentioned above, none of previous medical materials for use in the reconstruction of tissue defects satisfy many demands (1) to (7) described above at a high level.

The present invention has been made in light of the problems as described above. An object of the present invention is to provide a medical honeycomb structure that satisfies most of the demands (1) to (7) desired for medical materials and a method for producing the same, a medical tissue reconstruction bag incorporating the medical honeycomb structure or the like, and a molding die useful in the production of the medical honeycomb structure.

In the present invention, a starting material honeycomb structure, which is a raw material for the medical honeycomb structure, is also a honeycomb structure related to medicine, and is therefore defined as a medical honeycomb structure.

### Solution to Problem

The present inventors have conducted diligent studies and consequently completed the present invention by finding that a medical honeycomb structure lacking at least a portion of an outer peripheral side wall of a honeycomb structure comprising a plurality of through-holes extending in one direction, wherein sites lacking the outer peripheral side wall comprise a plurality of grooves, and comprise a plurality of planes including distant surfaces of groove side walls flanked by the grooves is a medical material that satisfies most of the demands (1) to (7) or/and satisfies these demands at a high level, and also by finding a related medical tissue reconstruction bag, molding die, and production method.

Specifically, the present invention is as follows (hereinafter, the following items [1] to [13] of the invention are also referred to as the present inventions [1] to [13]).
[1] A medical honeycomb structure lacking at least a portion of an outer peripheral side wall of a honeycomb structure comprising a plurality of through-holes extending in one direction, wherein sites lacking the outer peripheral side wall comprise a plurality of grooves, and comprise a plurality of planes including distant surfaces of groove side walls flanked by the grooves, with the proviso that a medical honeycomb structure wherein the sites lacking the outer peripheral side wall comprising grooves and through-hole inlets adjacent to the grooves is excluded.
[2] The medical honeycomb structure according to [1], wherein the medical honeycomb structure comprises a groove side wall having an undercut in a through-hole direction and/or in a direction perpendicular to a distant surface of a groove bottom wall.
[3] The medical honeycomb structure according to [1] or [2], wherein the medical honeycomb structure comprises a groove side wall that satisfies at least one of the following (A) to (C):
   (A) a value obtained by dividing a height of the groove side wall by a width of the groove side wall is 3 or less;
   (B) a value obtained by dividing the height of the groove side wall by the width of the groove is 0.8 or less; and
   (C) a value obtained by dividing the width of the groove side wall by the width of the groove is less than 1.
[4] The medical honeycomb structure according to any one of [1] to [3], wherein the medical honeycomb structure has a honeycomb aspect ratio obtained by dividing a maximum length of the through-hole or the groove by a major axis of a plane perpendicular to the through-hole of 3 or less, and an apparent volume of 1 × 10⁻⁷ m³ or smaller.
[5] A medical honeycomb structure molding die for use in production of a medical honeycomb structure lacking at least a portion of an outer peripheral side wall of a honeycomb structure comprising a plurality of through-holes extending in one direction, wherein sites lacking the outer peripheral side wall comprise a plurality of grooves, and comprise a plurality of planes including distant surfaces of groove side walls flanked by the grooves, wherein at least one outermost peripheral pin and a mask inner face are substantially in contact and/or are integrated with each other.
[6] A medical tissue reconstruction bag comprising a mesh section in at least one portion, wherein a mesh opening area is 2 × 10⁻⁹ m² or larger, and a value obtained by dividing a total opening area of the mesh by an area of the mesh section is 0.2 or more,
   comprising an introduction port for incorporation of a medical tissue reconstruction material, or incorporating a medical tissue reconstruction material without an introduction port, and
   satisfying at least one requirement selected from the group of (D) to (F):
   (D) having a mesh opening area of 2 × 10⁻⁵ m² or smaller, and comprising a structure that reduces enlargement of the bag associated with the incorporation of a material;
   (E) having a mesh opening area of 2 × 10⁻⁵ m² or smaller, and exhibiting a strain of 0.2 or more under a load of 10 N in an arbitrary direction; and
   (F) having a mesh opening area of 1.0 × 10⁻⁷ m² or smaller.
[7] A medical tissue reconstruction bag comprising a mesh section in at least one portion, wherein a mesh opening area is 2 × 10⁻⁹ m² or larger and 2 × 10⁻⁵ m² or smaller, and a value obtained by dividing a total opening area of the mesh by an area of the mesh section is 0.2 or more, and incorporating a carbonate apatite medical tissue reconstruction material.
[8] The medical tissue reconstruction bag according to [6] or [7], wherein the medical tissue reconstruction bag comprises a hole and/or a thread for fixing the medical tissue reconstruction bag to a tissue.
[9] The medical honeycomb structure according to any one of [1] to [4], wherein the medical honeycomb structure comprises at least one selected from the group consisting of carbonate apatite, apatite, tricalcium phosphate, octacalcium phosphate, calcium phosphate, calcium carbonate, calcium sulfate, calcium-containing glass, collagen, chitosan, polyglycolic acid, polylactic acid, poly(lactic-co-glycolic acid), and poly(lactideco-caprolactone) in composition.
[10] The medical tissue reconstruction bag according to any one of [6] to [8], wherein the medical tissue reconstruction material comprises at least one selected from the group consisting of carbonate apatite, apatite, tricalcium phosphate, octacalcium phosphate, calcium phosphate, calcium carbonate, calcium sulfate, calcium-containing glass, collagen, chitosan, polyglycolic acid, polylactic acid, poly(lactic-co-glycolic acid), and poly(lactideco-caprolactone) in composition.
[11] A method for producing a medical honeycomb structure, comprising a step of producing a medical honeycomb structure lacking at least a portion of an outer peripheral side wall of a honeycomb structure comprising a plurality of through-holes extending in one direction, wherein sites lacking the outer peripheral side wall comprise a plurality of grooves,
   the production step comprising a step of changing a ratio of an extrusion rate from a honeycomb molding die to a rate at which an extruded honeycomb structure is transferred so as to move away from the honeycomb molding die.
[12] A method for producing a medical honeycomb structure, comprising a step of producing a medical honeycomb structure lacking at least a portion of an outer peripheral side wall of a honeycomb structure comprising a plurality of through-holes extending in one direction, wherein sites lacking the outer peripheral side wall comprise a plurality of grooves,
   the production step comprising adding a polymer at least to a portion of the grooves at the sites lacking the outer peripheral side wall, and then processing the medical honeycomb structure, followed by removal of the polymer.
[13] A method for producing a medical honeycomb structure, comprising a step of heating and degreasing a layer of a plurality of honeycomb structures containing a polymer, wherein a powder and/or granules of inorganic matter are interposed between the honeycomb structures.

### Advantageous Effects of Invention

The medical honeycomb structure of the present invention satisfies most of the demands (1) to (7) and/or satisfies these demands at a high level, and can be widely used in the medical field or fields related to medicine, together with a related medical tissue reconstruction bag, molding die, and production method.

### Brief Description of Drawings

Figure 1 is a schematic view of a general honeycomb structure comprising an outer peripheral side wall that is not included in the scope of the present invention.
Figure 2 is a schematic view (one example) of the medical honeycomb structure of the present invention.
Figure 3 is a schematic view (one example) showing the relationship between a groove and components surrounding the groove on the most distant surface of the medical honeycomb structure of the present invention.
Figure 4 is a schematic view (one example) of through-holes and grooves in a general honeycomb structure comprising an outer peripheral side wall that is not included in the scope of the present invention, and in the medical honeycomb structure of the present invention.
Figure 5 is a schematic view (one example) of a general honeycomb structure comprising an outer peripheral side wall that is not included in the scope of the present invention, and the medical honeycomb structure of the present invention viewed from the through-hole direction.
Figure 6 is a schematic view (one example) of a groove side wall of the medical honeycomb structure of the present invention.
Figure 7 is a schematic view (one example) of a groove side wall of the medical honeycomb structure of the present invention.
Figure 8 is a schematic view (one example) of a groove side wall of the medical honeycomb structure of the present invention.
Figure 9 is a schematic view (one example) of a general honeycomb structure molding die that is not included in the scope of the present invention.
Figure 10 is a schematic view (one example) of the medical honeycomb structure molding die of the present invention.
Figure 11 is a schematic view (one example) of the medical tissue reconstruction bag of the present invention.
Figure 12 is an electron microscope photograph (SEM photograph) of the medical honeycomb structure of the present invention (Examples 1 and 2).
Figure 13 is an electron microscope photograph (SEM photograph) of the medical honeycomb structure of the present invention (Example 4).
Figure 14 is a pathological tissue image of the medical honeycomb structure of the present invention according to Example 4.
Figure 15 is an electron microscope photograph (SEM photograph) of a honeycomb structure that is not included in the scope of the present invention (Comparative Example 1).
Figure 16 is a µCT image of the medical tissue reconstruction bag of the present invention according to Example 6.

### Description of Embodiments

Hereinafter, the present invention will be described.

### <General honeycomb structure>

The honeycomb structure is a material having a honeycomb structure comprising a plurality of through-holes extending in one direction. The honeycomb structure may be regarded as a structure where polygonal hollow columns or round hollow columns are tightly arranged.

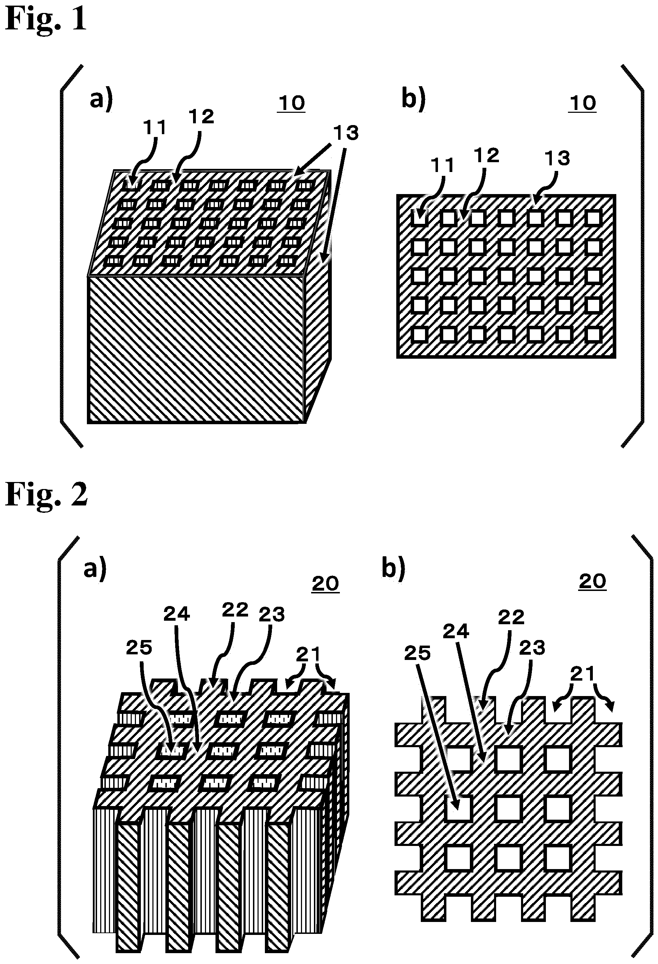

Here, a general honeycomb structure will be described with reference to Figure 1. Figure 1a) is a perspective view of a honeycomb structure, and Figure 1b) is a view of the honeycomb structure viewed from the extending direction of through-holes (in the through-hole direction). Honeycomb structure 10 is a columnar object comprising a plurality of through-holes 11 extending in one direction, partition walls 12 which section the through-holes from each other, and outer peripheral side wall 13. The outer peripheral side wall 13 is located on the outer peripheral side of the honeycomb structure and is a wall that faces the outside. The partition walls 12 and the outer peripheral side wall 13 are walls constituting the honeycomb structure. In order to distinguish therebetween, a wall located on the outer periphery of outermost peripheral through-holes 11 of the honeycomb structure is referred to as outer peripheral side wall 13 and thus distinguished from partition walls 12 which are the other walls.

### <Method for producing general honeycomb structure>

In general, the honeycomb structure is produced from a raw material through a honeycomb molding die using an extrusion molding machine or the like. For example, an organic binder-containing hydroxyapatite honeycomb structure having an outer peripheral side wall is produced by using a mixture of a hydroxyapatite powder and an organic binder as a raw material, and molding the mixture by, for example, a method disclosed in Japanese Patent No. 3405536 or Japanese Patent Laid-Open Publication No. 10-59784. The honeycomb structure is debindered to produce a hydroxyapatite honeycomb structure having an outer peripheral side wall.

Also, a collagen-containing carbonate apatite honeycomb structure comprising an outer peripheral side wall is produced by using a mixture of a carbonate apatite powder and collagen as a raw material, and extruding the mixture through a honeycomb molding die, followed by drying.

### <1 Basic conditions of present invention>

The present invention relates to a medical material. The medical material is a material for use in clinical practice or medical-related fields such as cell culture, and therefore differs in required properties from other materials.

The medical honeycomb structure of the present invention is a honeycomb structure having a specific structure useful in medicine. In the present invention, a honeycomb structure before degreasing for use in the production of the medical honeycomb structure for use in clinical practice and the like is also defined as a medical honeycomb structure.

A feature of the medical honeycomb structure of the present invention is to lack at least a portion of an outer peripheral side wall of a honeycomb structure comprising a plurality of through-holes extending in one direction, wherein the sites lacking the outer peripheral side wall comprise a plurality of grooves, and comprise a plurality of planes including distant surfaces of groove side walls flanked by the grooves.

In other words, in the medical honeycomb structure, the outer peripheral surface of a honeycomb structure comprising a plurality of through-holes extending in one direction comprises a plurality of grooves formed by a plurality of groove side walls extending in the through-hole direction, and the plurality of grooves include grooves that face different distant directions. Since the honeycomb structure lacks at least a portion of the outer peripheral side wall, a medical structure, wherein the outer peripheral surface of the structure comprising one through-hole extending in one direction comprises a plurality of grooves formed by a plurality of groove side walls extending in the through-hole direction, and the plurality of grooves include grooves that face different distant directions, and a medical structure, wherein the outer peripheral surface of the structure extending in one direction comprises a plurality of grooves formed by a plurality of groove side walls, and the plurality of grooves include grooves that face different distant directions are also included in the scope of the present invention.

However, a medical honeycomb structure wherein the sites lacking the outer peripheral side wall (outer peripheral surface) comprise grooves and through-hole inlets adjacent to the grooves is not included in the scope of the present invention. Specifically, the grooves on the outer peripheral surface of the medical honeycomb structure of the present invention are continuously formed from one end to the other end in the through-hole direction of the structure.

The conduction of tissues occurs only on the surface of a medical tissue reconstruction material. Therefore, the medical honeycomb structure comprising grooves on the surface is highly useful in tissue reconstruction surgery.

Here, medical honeycomb structure 20 of the present invention will be described with reference to Figures 2 to 8.

Figure 2a) is a perspective view of one example of the medical honeycomb structure of the present invention, and Figure 2b) is a view of the honeycomb structure viewed from the through-hole direction. The honeycomb structure lacks the whole of the outer peripheral side wall 13 of a general honeycomb structure that is not included in the scope of the present invention. The honeycomb structure lacking the outer peripheral side wall 13 comprises, on the outer peripheral side grooves 21, groove side walls 22 flanked by the grooves 21, and groove bottom walls 23 on the mesial side of the grooves 21.

The outer peripheral side wall 13 prevents the honeycomb structure from being peripherally spread and poorly extruded upon extrusion. The outer peripheral side wall also plays an important role in the mechanical strength and handling of the honeycomb structure. Thus, the medical honeycomb structure of the present invention lacking a portion or the whole of the outer peripheral side wall 13 is relatively difficult to produce and is inferior in mechanical strength or handling.

However, the medical honeycomb structure of the present invention satisfies most of the demands (1) to (7), and/or satisfies these demands at a high level for tissue reconstruction surgery. The demands (1) to (4), the level of which is high, will be mainly described.

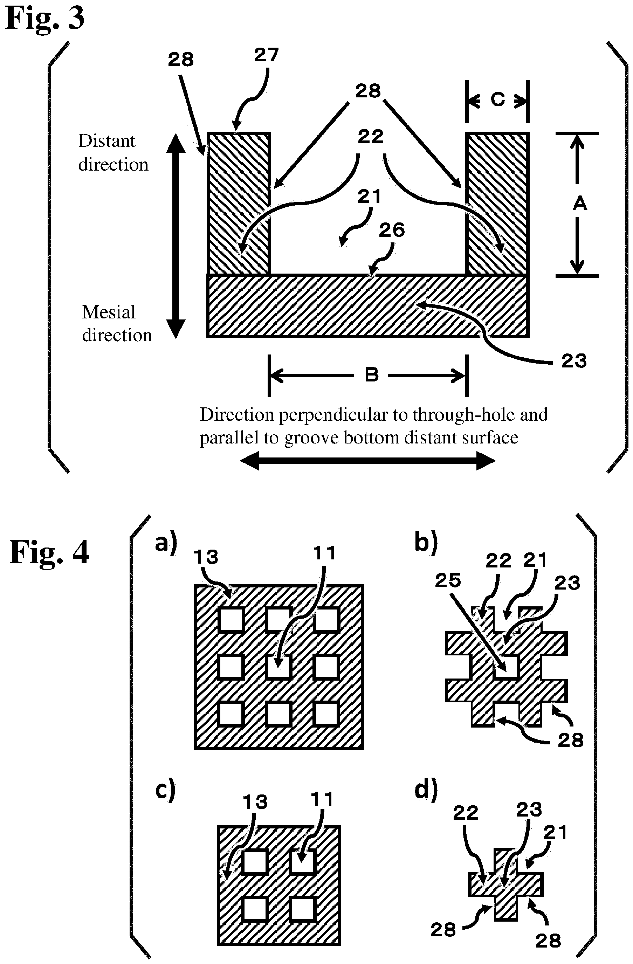

The components surrounding the groove 21 will be described with reference to Figure 3. The groove 21 is a space surrounded by groove bottom wall 23 and groove side wall 22 which runs in the distant direction from the groove bottom wall 23. The groove 21 may be a space surrounded by two groove side walls 22. The surface in the distant direction of the groove bottom wall 23 is referred to as groove bottom wall distant surface 26. The surface in the distant direction of the groove side wall 22 is referred to as groove side wall distant surface 27. The side surfaces of the groove side wall 22 are referred to as groove side wall side surfaces 28. The groove side wall side surfaces 28 are located on both sides of the groove side wall 22. In the present invention [1], the "distant surfaces of groove side walls flanked by the grooves" are groove side wall distant surfaces 27.

The distant direction and the mesial direction refer to the distant direction and the mesial direction viewed from the center of the honeycomb structure. Specifically, the distant direction is a direction that is far from the center of the honeycomb structure, and the mesial direction is a direction that is near from the center of the honeycomb structure.

The height (indicated by A of Figure 3) of the groove side wall is the length in the mesial and distant directions of the groove side wall 22, and does not include the thickness of the groove bottom wall 23. The width (indicated by B of Figure 3) of the groove is the interval between two groove side walls 22 that face each other.

The width (indicated by C of Figure 3) of the groove side wall is the width (thickness) in a direction perpendicular to the through-hole, of the groove side wall on a surface where the groove side wall 22 is in contact with the groove bottom wall 23. In a medical honeycomb structure that differs in the height or width of the groove side wall depending on sites, the height or width, if not defined, of the groove side wall is a height or a width at a site that attains the maximum value.

The through-hole will be described with reference to Figure 4 when the honeycomb structure is viewed from the through-hole direction. Figures 4a) and 4c) each show a honeycomb structure comprising nine or four through-holes 11 extending in one direction. This honeycomb structure is not included in the scope of the present invention, because it comprises outer peripheral side wall 13 on the entire surface. Figures 4b) and 4d) each show the medical honeycomb structure of the present invention lacking the outer peripheral side wall 13 of the honeycomb structure, wherein the sites lacking the outer peripheral side wall 13 comprise a plurality of grooves 21. In general, the honeycomb structure is regarded as a structure comprising a plurality of through-holes extending in one direction. In light of the nature of the present invention, such a structure lacking the outer peripheral side wall 13 of the honeycomb structure, wherein the sites lacking the outer peripheral side wall 13 comprise grooves 21 is also defined as the honeycomb structure even if the structure has one through-hole or no through-hole. In the present invention, when a through-hole is curved, a direction that connects both ends of the through-hole is referred to as the through-hole direction. In a structure having no through-hole, the direction of grooves is defined as the through-hole direction.

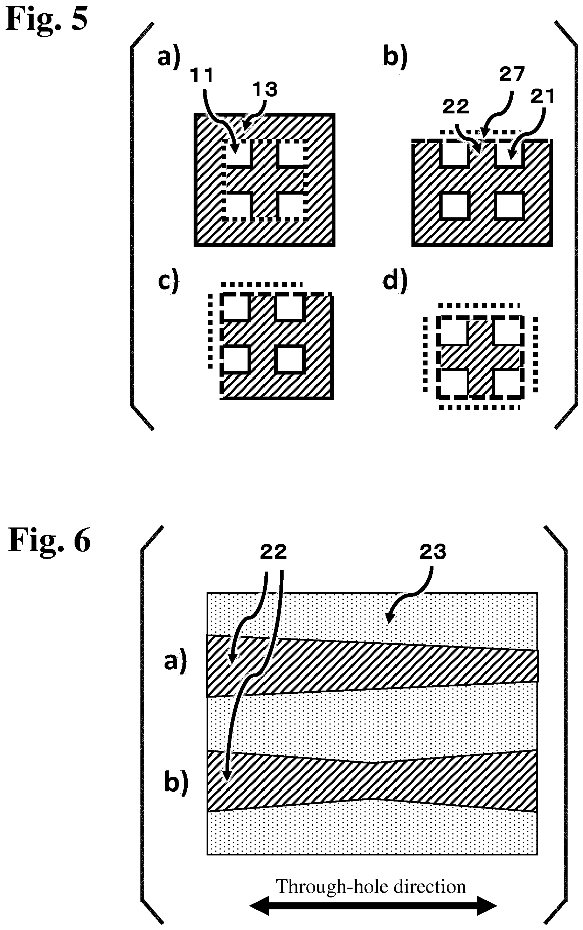

The essential requirement that the sites lacking the outer peripheral side wall "comprise a plurality of planes including distant surfaces of groove side walls flanked by the grooves" in the medical honeycomb structure of the present invention will be described with reference to Figure 5, where the honeycomb structure is viewed from the through-hole direction. Figure 5a) shows a honeycomb structure that is not included in the scope of the present invention because of comprising outer peripheral side wall 13 on the entire surface. A honeycomb structure comprising groove 21 and groove side wall 22 shown in Figure 5b) can be produced by removing one surface of the outer peripheral side wall 13 from the honeycomb structure of Figure 5a). A plane (virtual plane) including groove side wall distant surface 27 serving as the upper surface of the groove side wall is indicated by a broken line in the drawing. The honeycomb structure of Figure 5b) having only one such plane is a honeycomb structure that is not included in the scope of the present invention. On the other hand, a honeycomb structure of Figure 5c) or 5d) comprising two or four groove side wall distant surfaces 27 is the medical honeycomb structure of the present invention. Specifically, the medical honeycomb structure of the present invention can be regarded as comprising groove side wall distant surfaces that face two or more different distant directions. A plurality of such planes need to exist under conditions where no stress is applied to the honeycomb structure.

The presence of the plurality of planes allows the honeycomb structure to satisfy the demands (1) to (4) at a higher level. This is because the grooves and the groove side walls of the medical honeycomb structure highly bind to tissue defects. In the medical honeycomb structure, when the ratio of grooves is larger, it facilitates binding to tissue defects.

A value obtained by dividing the area of a virtual surface that connects the sites lacking the outer peripheral side wall by the area of the inside surface of the outer peripheral side wall is defined as a lacking outer peripheral side wall ratio. The ratio will be described with reference to Figure 5. In Figure 5a), the inside surface of the outer peripheral side wall is indicated by a dotted line. In Figures 5b), 5c), and 5d), the length of the virtual surface that connects the sites lacking the outer peripheral side wall is indicated by a dotted line. In order to distinguish from the broken line used in the description about the plane including the upper surface of the groove side wall, the dotted line is described with a slight shift in the distant direction from the virtual surface that connects the sites lacking the outer peripheral side wall. The ratios in Figure 5b), 5c), or 5d) are 0.25, 0.5, and 1.0, respectively. The ratio is preferably 0.3 or more, more preferably 0.6 or more, further preferably 0.9 or more, ideally 1.0, i.e., a complete loss of the outer peripheral side wall, for satisfying the demands (1) to (4) at a higher level.

A medical tissue reconstruction material in the form of granules satisfies a part of the demand (1), i.e., can conveniently reconstruct a tissue defect. Specifically, the implantation of a block in a tissue defect requires morphogenesis, whereas in case of granules, it suffices to simply fill a tissue defect with the medical honeycomb structure. From this viewpoint, the apparent volume of the medical honeycomb structure may be preferably 2 × 10⁻⁷ m³ or smaller, may be more preferably 3 × 10⁻⁸ m³ or smaller, may be further preferably 1 × 10⁻⁸ m³ or smaller, and may be particularly preferably 1 × 10⁻⁹ m³ or smaller.

Simple granules easily move away from a tissue defect. However, in case of a medical honeycomb structure having a small apparent volume, it is less likely to move away from a tissue defect because a given mating force is generated between the medical honeycomb structures through the grooves 21.

The apparent volume described in the present invention is the volume of a section surrounded by the outer periphery of the structure and is defined as the total sum of the volume of the material object itself of the structure, the volumes of pores within the structure, and the surface groove volumes of the structure. The groove volume is a volume that surrounds the groove 21. Specifically, the groove volume is the volume of a section surrounded by two groove side wall side surfaces 28 flanking the groove 21, groove bottom distant surface 26, a plane passing through two intersections between the groove side wall side surfaces 28 flanking the groove 21 and groove side wall distant surfaces 27, and planes substantially perpendicular to the groove direction at both ends of the groove 21.

In the medical honeycomb structure of the present invention, the form of the through-hole is not particularly limited, and a polygonal prismatic or cylindrical through-hole can be used without limitations. However, a polygonal prismatic through-hole is more preferred than a cylindrical one in view of the balance between the ability to form tissues and the cost. A substantially quadrangular prismatic through-hole may be preferred.

### <2 Groove side wall having undercut>

The medical honeycomb structure according to the present invention [2] as the medical honeycomb structure of the present invention satisfies the demands (1) to (5) at a higher level because groove side wall 22 has an undercut. The undercut is a recess that is not visible when the structure is viewed from a specific direction. The undercut prevents the structure from migrating in a direction opposite to the specific direction when tissues are formed surrounding the structure, or enhances mechanical strength. Figure 6 is a view of groove side wall 22 of the medical honeycomb structure 20 viewed from the distant direction. The groove side wall 22 of Figure 6a) has an undercut when viewed from the leftward direction (with respect to the leftward direction) of the drawing. The undercut prevents the medical honeycomb structure from migrating in the rightward direction of the drawing. On the other hand, the groove side wall 22 has no undercut when viewed from the rightward direction (with respect to the rightward direction) of the drawing. Hence, the medical honeycomb structure cannot be prevented from migrating in the leftward direction of the drawing. On the other hand, the groove side wall 22 of Figure 6b) has an undercut when viewed from both left and right sides of the drawing. Specifically, the groove side wall 22 has an undercut with respect to both directions of the through-hole. Hence, the groove side wall of Figure 6b) can prevent the medical honeycomb structure from migrating in both directions of the through-hole. The groove side wall of the medical honeycomb structure of the present invention is preferably a groove side wall having an undercut with respect to at least one direction of the through-hole, more preferably a groove side wall having an undercut with respect to both directions of the through-hole.

The medical honeycomb structure comprising the groove side wall having an undercut satisfies the demands (1) to (5) at a higher level. Medical honeycomb structure 20 comprising the following specific groove side wall 22 is preferred.

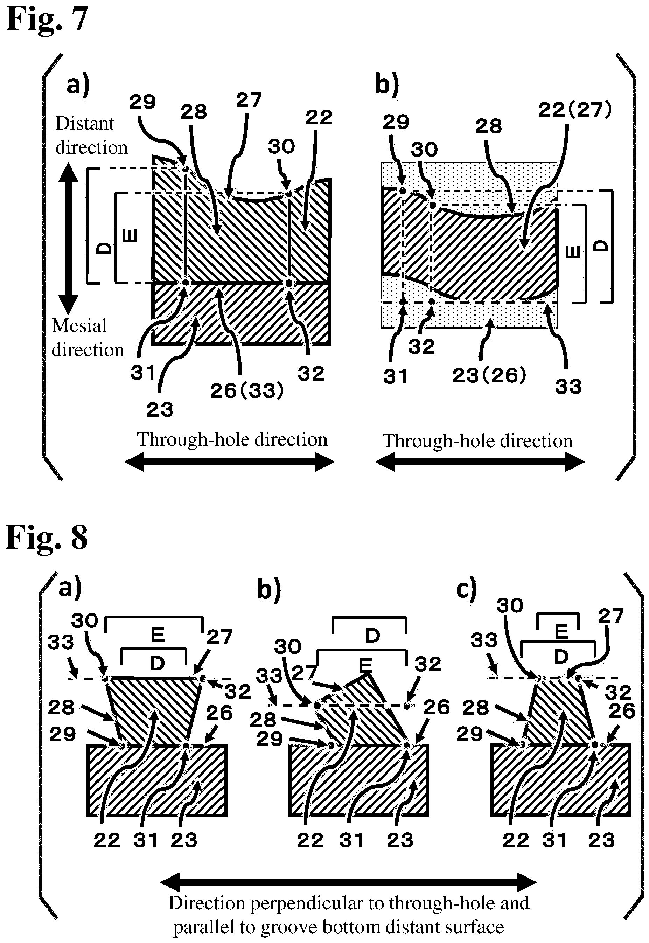

The specific groove side wall 22 having an undercut with respect to the through-hole direction will be described with reference to Figure 7a) when groove side wall side surface 28 which is the side surface of the groove side wall 22 of the medical honeycomb structure of the present invention is viewed from a direction perpendicular to the through-hole, and Figure 7b) when groove bottom wall distant surface 26 which is the distant surface of groove bottom wall 23 is viewed therefrom. The groove bottom wall 23 comprises the groove side wall 22 in the distant direction. The rightward and leftward direction of Figure 7 is the through-hole direction. In Figure 7a), the distant direction is positioned on the upper side, and the mesial direction is positioned on the lower side.

First, specific groove side wall 22 with groove side wall distant surface 27 having an undercut with respect to the through-hole direction will be described with reference to Figure 7a). In such specific groove side wall, an arbitrary point on the line of intersection between groove side wall side surface 28 and groove bottom wall distant surface 26 is defined as base point 29; a point that is located on the same line of intersection and is different from the base point 29 is defined as another point 30; and points at which perpendicular lines drawn from the base point 29 and the another point 30 to the groove bottom wall distant surface 26 intersect with the groove bottom wall distant surface 26 are defined as counter base point 31 and another counter point 32, respectively.

Next, specific groove side wall 22 with groove side wall side surface 28 having an undercut with respect to the through-hole direction will be described with reference to Figure 7b). In such specific groove side wall, an arbitrary point on the line of intersection between the groove side wall side surface 28 and groove side wall distant surface 27 is defined as base point 29, and a point that is located on this line of intersection and is different from the base point 29 is defined as another point 30. A plane that is parallel to a plane parallel to the through-hole direction, including a perpendicular line drawn from the base point 29 to groove bottom wall distant surface 26, and passes through the point of tangency on the line of intersection between groove side wall side surface 26 on the side where the base point 27 is absent, and the groove side wall distant surface 25 is defined as reference plane 33. When the groove side wall is, for example, in a triangular prismatic form, having no groove side wall distant surface 27, the base point 29 and the another point 30 are set to points on a line formed by groove side wall side surface 28 located farthest from the groove bottom wall distant surface 26. Points at which perpendicular lines drawn from the base point 29 and the another point 30 to the reference plane 33 intersect with the reference plane 33 are defined as counter base point 31 and another counter point 32, respectively.

Specific groove side wall 22 having an undercut with respect to a direction perpendicular to groove bottom wall distant surface 26 will be described with reference to Figure 8 where the groove side wall 22 of the medical honeycomb structure of the present invention is viewed from the through-hole direction. The rightward and leftward direction of Figure 8 is a direction perpendicular to the through-hole and parallel to the groove bottom distant surface 26. The distant direction is positioned on the upper side, and the mesial direction is positioned on the lower side.

First, specific groove side wall 22 having an undercut with respect to a mesial direction perpendicular to groove bottom wall distant surface 26 will be described with reference to Figure 8a). In such specific groove side wall, an arbitrary point on the line of intersection between groove side wall side surface 28 on one side and the groove bottom wall distant surface 26 is defined as base point 29. A point that is located on a plane (which passes through the base point 29) perpendicular to the through-hole direction, and is the intersection between the groove bottom wall distant surface 26 and groove side wall side surface 28 on the other side is defined as counter base point 31; the intersection between the groove side wall side surface 28 on the same side with the base point 29 and groove side wall distant surface 27 is defined as another point 30; and the intersection between the groove side wall side surface 28 that passes through the counter base point 31 and the groove side wall distant surface 27 is defined as another counter point 32.

Next, specific groove side wall 22 that is inclined and has an undercut with respect to a mesial direction perpendicular to groove bottom wall distant surface 26 will be described with reference to Figure 8b). In such specific groove side wall, an arbitrary point on the line of intersection between groove side wall side surface 28 with a dihedral angle of less than 90°C against the groove bottom wall distant surface, and the groove bottom wall distant surface 26 is defined as base point 29. A point that is located on a plane (which passes through the base point 29) perpendicular to the through-hole direction, and is the intersection between groove side wall side surface 28 on the side opposite to the groove side wall side surface 28 that passes through the base point 29, and the groove bottom wall distant surface 26 is defined as counter base point 31. The intersection among the groove side wall side surface 28 on the base point 29 side, groove side wall distant surface 27, and the plane (which passes through the base point 29) perpendicular to the through-hole direction is defined as another point 30. A plane that is parallel to the groove bottom wall distant surface 26 and passes through the another point 30 is defined as reference plane 33, and the intersection between a perpendicular line drawn from the counter base point 31 to the reference plane 33, and the reference plane 33 is defined as another counter point 32.

Next, specific groove side wall 22 having an undercut with respect to a distant direction perpendicular to groove bottom wall distant surface 26 will be described with reference to Figure 8c). In such specific groove side wall, an arbitrary point on the line of intersection between groove side wall side surface 28 on one side and the groove bottom wall distant surface 26 is defined as base point 29. A point that is located on a plane (which passes through the base point 29) perpendicular to the through-hole direction, and is the intersection between groove side wall side surface 28 on the side opposite to the base point 29, and the groove bottom wall distant surface 26 is defined as counter base point 31; the intersection between the groove side wall side surface 28 on the base point 29 side and groove side wall distant surface 27 is defined as another point 30; and the intersection between the groove side wall side surface 28 on the counter base point 31 side and the groove side wall distant surface 27 is defined as another counter point 32.

In these specific groove side walls 22, the length from the base point 29 to the counter base point 31 is defined as a base point length (indicated by D in Figures 7 and 8), and the length from the another point 30 to the another counter point 32 is defined as another point length (indicated by E in Figures 7 and 8). Groove side wall 22 having a difference between the base point length and the another point length has an undercut with respect to the through-hole direction or/and a direction perpendicular to groove bottom wall distant surface 26. Of the base point length (D) which is the length from the base point 29 to the counter base point 31 and the another point length (E) which is the length from the another point 30 to the another counter point 32, the value obtained by dividing the larger length by the smaller length is defined as an undercut index. When value of the undercut index is larger, the undercut is larger, which is preferable. The undercut index is preferably 1.05 or more, more preferably 1.1 or more, further preferably 1.3 or more.

When the number of groove side walls having an undercut is larger, the effect is enhanced. Therefore, in the honeycomb structure of the present invention, it is preferable that there is 10% or more of the groove side wall 22 having an undercut, more preferably 30% or more, further preferably 50% or more, particularly preferably 70% or more, most preferably 90% or more, with respect to all the groove side walls of the honeycomb structure. Ideally, all the groove side walls have an undercut.

### <3 Height and width of groove side wall>

Grooves 21 or/and groove side walls 22 play an important role in satisfying the demands (1) to (5) and however, may be susceptible to breakage.

Figure 3 shows the width and height of groove side wall 22 and the width of groove 21. An important factor for groove side wall 22 to be not broken is the relative height of the groove side wall 22. A value obtained by dividing the height (indicated by A of Figure 3) of the groove side wall 22 by the width (indicated by C of Figure 3) of the groove side wall 22 is preferably 3 or less. The value is more preferably 2.5 or less, further preferably 2.0 or less. A value obtained by dividing the height (indicated by A of Figure 3) of the groove side wall by the width (indicated by B of Figure 3) of the groove 21 is preferably 0.8 or less. The value is more preferably 0.7 or less, further preferably 0.6 or less.

Meanwhile, the width (indicated by B of Figure 3) of the groove 21 and the width (indicated by C of Figure 3) of the groove side wall 22 play an important role in fixing medical honeycomb structures to each other. The fixation of medical honeycomb structures to each other renders their groove side walls 22 insusceptible to breakage. When the width of the groove 21 is longer than the width of the groove side wall 22, i.e., when the value obtained by dividing the width of the groove side wall 22 by the width of the groove 21 is less than 1, the groove side wall 22 of a medical honeycomb structure may enter into the groove 21 of another medical honeycomb structure and fixed. Hence, the value is preferably less than 1, more preferably 0.8 or less, further preferably 0.6 or less.

### <4 Medical honeycomb structure having specific apparent volume and honeycomb aspect ratio>

The medical honeycomb structure according to the present invention [4] satisfies the conditions (1) to (4) at a high level.

In the present invention, a value obtained by dividing the maximum length of the through-hole or the groove by the maximum length of a plane perpendicular to the through-hole is defined as a honeycomb aspect ratio. An apparent volume of 1 × 10⁻⁷ m³ or smaller and a honeycomb aspect ratio of 3 or less facilitate filling a tissue defect with the honeycomb structure and therefore permit an easy surgery. Such a honeycomb structure has a high packing density in a tissue defect and therefore easily reconstructs tissue defects. Particularly, in the case of a medical honeycomb structure that satisfies the demand (C), honeycomb structures are fixed to each other through grooves 21 and groove side walls 22 and are therefore less likely to move away from a tissue defect. A crushed product of the honeycomb structure is also effective to some extent and however, is excluded from the present invention [4] because its grooves are susceptible to breakage and are inferior in the ability to fix the honeycomb structure.

The apparent volume is preferably 1 × 10⁻⁷ m³ or smaller, more preferably 7 × 10⁻⁷ m³ or smaller, further preferably 3 × 10⁻⁷ m³ or smaller, in relation to a packing density. The honeycomb aspect ratio is preferably 3 or less, more preferably 2 or less, further preferably 1.5 or less, in relation to a packing density.

### <5 Medical honeycomb structure molding die>

A general honeycomb molding die has been proposed in Japanese Patent Laid-Open Publication Nos. 7-24817 and 11-320526, etc.

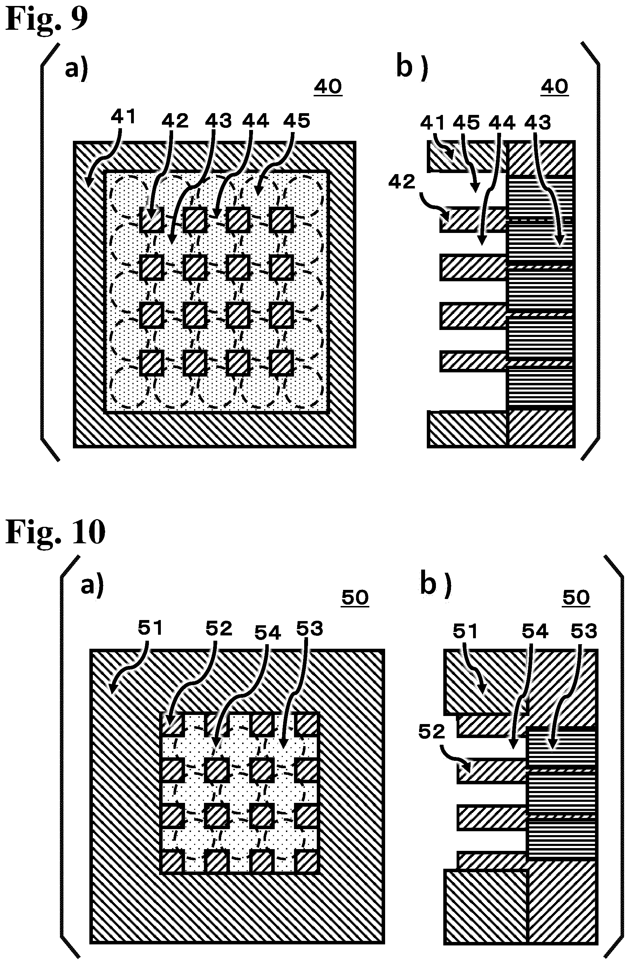

Figure 9 is one example of a schematic view of general honeycomb molding die 40 that is not included in the scope of the present invention. Figure 9a) is a schematic view of the honeycomb molding die viewed from the direction of extrusion of a raw material, and Figure 9b) is a cross-sectional view of a honeycomb structure on a plane in the direction of extrusion of a raw material, including vertical straight lines that pass through pins 42 of Figure 9a).

The honeycomb molding die 40 of Figure 9 comprises mask 41 detachable from the main body. Although the mask 41 does not have to be detachable from the main body, a detachable mask permits easy processing.

The honeycomb molding die comprises raw material supply holes 43 on the side where a raw material is supplied. Pins 42 are located in the direction of extrusion of a raw material from the raw material supply holes. Mask 41 is located at a site distant from the outer peripheries of the pins. The spaces between a plurality of pins are referred to as inter-pin grooves 44. The space between outermost peripheral pins 42 and the mask 41 is referred to as pin outer periphery 45.

A raw material is supplied from the raw material supply holes 43 using an extrusion molding machine or the like. The raw material supplied from the raw material supply holes 43 is supplied to the inter-pin grooves 44 and the pin outer periphery 45. The pins 42 form through-holes 11, and portions of the raw material supplied from the different raw material supply holes 43 bind to each other in the inter-pin grooves 44 and the pin outer periphery 45 to form partition walls 12 and outer peripheral side wall 13. As a result, through-holes 11, partition walls 12, and outer peripheral side wall 13 are formed to produce honeycomb structure 10 having an outer peripheral side wall.

A feature of the medical honeycomb structure of the present invention is to lack at least a portion of the outer peripheral side wall, wherein the sites lacking the outer peripheral side wall comprise a plurality of grooves.

For producing the medical honeycomb structure, a medical honeycomb structure molding die comprising at least one outermost peripheral pin and a mask inner face substantially contacting and/or integrated with each other may be useful.

Figure 10 shows one example of medical honeycomb structure molding die 50 of the present invention. In Figure 10, all outermost peripheral pins 52 substantially contact the inner face of mask 51.

Figure 10a) is a schematic view of the mold viewed from the direction of extrusion of a raw material, and Figure 10b) is a cross-sectional view of a plane in the direction of extrusion of a raw material, including vertical straight lines that pass through pins 52.

In such molding die, pin outer periphery 45 is substantially absent because the outermost peripheral pins 52 substantially contact the inner face of the mask 51. Hence, a raw material supplied from raw material supply holes 53 is supplied only to inter-pin grooves 54. Portions of the raw material supplied from the different raw material supply holes 53 bind to each other in the inter-pin grooves 54 to form through-holes 25, partition walls 24, groove bottom walls 23, and groove side walls 22. As a result, a medical honeycomb structure lacking an outer peripheral side wall, wherein the sites lacking the outer peripheral side wall comprise grooves 21, is produced.

The mask 51 is often produced being separated from the main body of the molding die from the viewpoint of processing accuracy, etc. Provided that the mask 51 is integrated with the main body, the mask 51 and the pins 52 in this structure are completely continuous. Such a structure is ideal because there is no gap between the inner face of the mask 51 and the pins 52.

In the present invention, the phrase "pin(s) and mask inner face substantially contact each other" means that the interval between the pin 52 and the inner face of the mask 51 is 10% or less of the interval between the pin and the pin, when the pins are viewed from the direction of extrusion of a raw material. This is because a when the interval between the pins and the mask is smaller, the pin outer periphery 45 formed between the pins and the mask is decreased, so that a raw material is less likely to enter the site. The value needs to be 10% or less and is preferably 8% or less, more preferably 6% or less, further preferably 4% or less. Ideally, the pins and the mask completely contact each other.

It is preferred that at least one outermost peripheral pin and a mask inner face are substantially contacting each other. When the number of grooves 21 is large, the honeycomb structure can satisfy the demands (1) to (4) at a higher level. Therefore, the percentage of the pin substantially contacting the mask inner face among the outermost peripheral pins is more preferably 40% or more, further preferably 70% or more, and ideally 100%.

### <6 Medical tissue reconstruction bag incorporating medical tissue reconstruction material>

For example, a medical honeycomb structure having a small apparent volume is a medical tissue reconstruction material that satisfies the demands (1) to (4) at a high level, while it easily moves away from a tissue defect as compared with a block material. Also, such a material is difficult to apply to extracoronal tissue reconstruction surgery. In this case, the present invention [6] is useful.

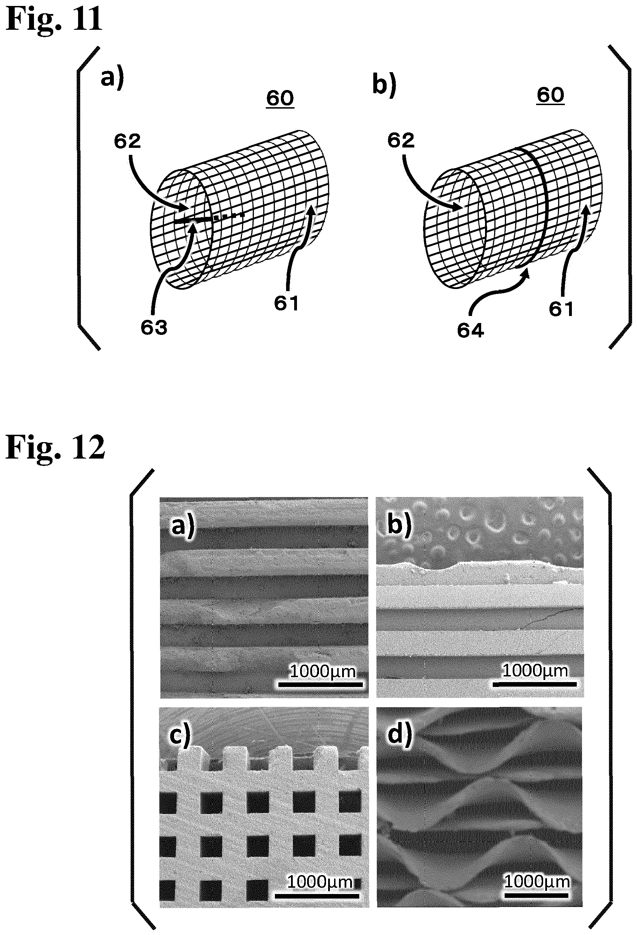

The present invention [6] will be described with reference to Figure 11. Medical tissue reconstruction bag 60 is a bag at least partially comprising a mesh section. The medical tissue reconstruction bag 60 of Figure 11 is a bag comprising a mesh section on the entire surface. The mesh section is a site of a mesh material woven into a netlike pattern and constitutes a portion or the whole of the bag. The mesh section comprises, in the net, mesh holes 61 serving as a communication section between the inside and the outside. The mesh opening area of the mesh holes 61 needs to be 2 × 10⁻⁹ m² or larger. This is an area necessary for the conduction of tissues from existing tissues into a medical tissue reconstruction material or the like incorporated in the medical tissue reconstruction bag 60. A thick mesh material having a small mesh opening area cannot secure a communication area sufficient for tissue conduction. Therefore, a value obtained by dividing the total opening area of the mesh holes 61 by the area of the mesh section needs to be 0.2 or more. The value is preferably 0.4 or more, more preferably 0.6 or more, further preferably 0.8 or more, from the viewpoint of efficient tissue conduction. The major axis of a plane perpendicular to the length direction of the mesh material is preferably 2 × 10⁻⁴ m or smaller, more preferably 1.5 × 10⁻⁴ m or smaller, further preferably 1 × 10⁻⁴ m or smaller. When the mesh bag exhibits stretchability, the mesh opening area of the mesh holes 61 and the value obtained by dividing the total opening area of the mesh holes 61 by the area of the mesh bag are measured in an arbitrary stretched state.

Introduction port 62 is used for allowing the medical tissue reconstruction bag 60 to incorporate a medical tissue reconstruction material or tissues. For example, a medical tissue reconstruction material such as a medical honeycomb structure having a small apparent volume is incorporated in the medical tissue reconstruction bag 60, and the introduction port 62 is closed. A medical tissue reconstruction bag incorporating a medical tissue reconstruction material and having no such introduction port 62 is also useful.

The medical tissue reconstruction bag incorporating the medical tissue reconstruction material with the introduction port 62 closed, or the medical tissue reconstruction bag incorporating the medical tissue reconstruction material and having no such introduction port 62 is fixed to a tissue defect. Such surgery attains a procedure of an extracoronal tissue reconstruction surgery such as vertical ridge augmentation, horizontal ridge augmentation, or saddle grafting, for example, by bone conduction from existing bones to the surface of the structure and the inside of the through-holes through the mesh holes 61 of the bag.

The material of the medical tissue reconstruction bag 60 is not particularly limited and may be preferably nylon, polyethylene, polypropylene, polystyrene, silicone, polyester, polyethylene terephthalate, polyurethane, titanium, titanium alloy, stainless steel, Co-Cr, Ni-Cr alloy, or the like from the viewpoint of tissue affinity or may be preferably a polymer or a copolymer of lactic acid, glycolic acid, caprolactone, lactide, or dioxane, or Mg alloy from the viewpoint of bioabsorbability.

In tissue reconstruction surgery, the functioning of the medical tissue reconstruction bag 60 requires a high level of mutual contact between contents such as the incorporated medical tissue reconstruction material. Tissues are conducted only to medical tissue reconstruction material surface, and tissue conduction is extremely worsen in the presence of gaps between contents such as the medical tissue reconstruction material. Also, tissue conduction is extremely worsen when the medical tissue reconstruction material is shaken. Hence, the medical tissue reconstruction bag 60 needs to further satisfy at least one requirement selected from the group of (D) to (F) which differs depending on tissue reconstruction surgery.

Medical tissue reconstruction bag 60 that satisfies the requirement (D) is useful for a procedure of a tissue reconstruction surgery on a case with a relatively large tissue defect. When the tissue defect area is large, the area of the bag also becomes larger, and the central part is enlarged by the incorporation of a medical tissue reconstruction material. Therefore, the form of the medical tissue reconstruction bag 60 is difficult to suit to the form of tissue defects. When the amount of the medical tissue reconstruction material to be incorporated is decreased, it cannot insure a high level of contact between contents such as the incorporated medical tissue reconstruction material. Hence, a structure that reduces the enlargement of the bag associated with the incorporation of a material is useful. The structure that reduces the enlargement of the bag associated with the incorporation of a material is, for example, a structure comprising inside string 63 for reduction in bag enlargement which connects two inside locations of the medical tissue reconstruction bag 60, as shown in Figure 11a). Another example thereof is a structure comprising surface string 64 for reduction in bag enlargement in the medical tissue reconstruction bag 60, as shown in Figure 11b). The surface string 64 for reduction in bag enlargement is useful when the medical tissue reconstruction bag 60 exhibits stretchability. The spring modulus of the surface string 64 for reduction in bag enlargement needs to be smaller than that of the surrounding bag. The structure that reduces the enlargement of the bag associated with the incorporation of a material can reduce increase in the volume of the bag. The ratio of the volume of the bag having the structure when the bag is fully filled with a material to the volume of the bag comprising no such structure when the bag is fully filled with a material may be preferably 0.9 or less, more preferably 0.8 or less, further preferably 0.6 or less.

The medical tissue reconstruction bag 60 comprises mesh holes 61. The mesh opening area of the mesh holes 61 needs to be 2 × 10⁻⁵ m² or smaller from the viewpoint of tissue conduction and the incorporation of a medical tissue reconstruction material. The area may be preferably 3 × 10⁻⁶ m² or smaller, more preferably 8 × 10⁻⁷ m² or smaller, further preferably 3 × 10⁻⁷ m² or smaller, depending on regenerated tissues.

Medical tissue reconstruction bag 60 that satisfies the requirement (E) is useful from the viewpoint of a high level of mutual contact between contents such as a medical tissue reconstruction material. Medical tissue reconstruction bag 60 that exhibits stretchability in every direction can densely incorporate a medical tissue reconstruction material. Specifically, a medical tissue reconstruction material is incorporated in the mesh material in an elongated state so that compressive stress acts on the medical tissue reconstruction material. The medical tissue reconstruction bag 60 needs to exhibit a strain of 0.2 or more under a load of 10 N in an arbitrary direction. Medical tissue reconstruction bag 60 having, for example, a length of 2 cm in the longitudinal direction needs to elongate to 2.4 cm or more under a load of 10 N in the longitudinal direction. The strain needs to be 0.2 or more and may be preferably 0.3 or more, more preferably 0.4 or more. The mesh opening area of the mesh holes 61 is the same as in the requirement (D).

Medical tissue reconstruction bag 60 that satisfies the requirement (F) is useful when a medical tissue reconstruction material having a small apparent volume is used. Tissues are conducted only to the surface of a medical tissue reconstruction material and are not conducted to a space in the medical tissue reconstruction material. Therefore, the medical tissue reconstruction bag 60 may preferably incorporate a medical tissue reconstruction material having a large apparent surface area per volume because of the small volume. The incorporation of such a medical tissue reconstruction material requires a small mesh opening area of mesh holes 61. The area needs to be 1 × 10⁻⁷ m² or smaller. The area may be more preferably 5 × 10⁻⁸ m² or smaller.

### <7 Medical tissue reconstruction bag incorporating carbonate apatite medical tissue reconstruction material>

Medical tissue reconstruction bag 60 incorporating a medical tissue reconstruction material undergoes tissue conduction to the medical tissue reconstruction material from existing tissues through mesh holes 61. Thus, the prognosis of a tissue reconstruction surgery depends on the medical tissue reconstruction bag 60 and the medical tissue reconstruction material. The medical tissue reconstruction bag 60 according to the present invention [8] incorporating a carbonate apatite medical tissue reconstruction material which exhibits markedly high bone conductivity is useful in bone reconstruction surgery. The markedly high bone conductivity of carbonate apatite is presumably due to bones composed mainly of carbonate apatite as an inorganic component, though a detailed mechanism underlying this is unknown. The medical tissue reconstruction bag 60 comprises mesh holes 61 having an area of 2 × 10⁻⁹ m² or larger and 2 × 10⁻⁵ m² or smaller. The area of the mesh holes 61 may be preferably 3 × 10⁻⁶ m² or smaller, more preferably 8 × 10⁻⁷ m² or smaller, further preferably 3 × 10⁻⁷ m² or smaller, depending on regenerated tissues. In the medical tissue reconstruction bag 60, the value obtained by dividing the total opening area of the mesh holes 61 by the area of the mesh bag, the major axis of a plane perpendicular to the length direction of the mesh material, the material, etc. are the same as in the description of the present invention [6].

The carbonate apatite of the present invention is apatite containing a carbonate group in the apatite structure of calcium phosphate apatite, as described in International Publication No. WO 2018/074429. The carbonate apatite refers to apatite in which a portion or the whole of phosphoric acid or hydroxy groups of apatite is substituted by a carbonate group. In the present invention, apatite having a carbonate group content of 0.1% by mass or more is defined as carbonate apatite. The carbonate group content is preferably 1% by mass or more, more preferably 3% by mass or more, further preferably 5% by mass or more, from the viewpoint of similarity to bones.

### <8 Medical tissue reconstruction bag having fixing structure>

When the medical tissue reconstruction bag incorporating a medical tissue reconstruction material is fixed to a tissue defect, bones are conducted, for example, from existing bones to the surface of the medical tissue reconstruction material through mesh holes 61 of the bag. The medical tissue reconstruction material needs to be in tight contact with the existing bones as much as possible from the viewpoint of bone conduction. From this viewpoint, the medical tissue reconstruction bag 60 according to the present invention [8] may be preferred. The hole is useful in fixing the medical tissue reconstruction bag to tissues using a screw or the like. Also, the medical tissue reconstruction bag comprising a thread can be fixed to tissues by the thread.

### <9 Particularly preferred composition of medical honeycomb structure>

The composition of the medical honeycomb structure of the present invention is not particularly limited and preferably comprises ceramic or a polymer material excellent in tissue affinity. For use in bone tissue reconstruction surgery, ceramic comprising at least one member selected from the group consisting of carbonate apatite, apatite, tricalcium phosphate, octacalcium phosphate, calcium phosphate, calcium carbonate, calcium sulfate and calcium-containing glass in composition is particularly preferred as composition contained in the medical honeycomb structure of the present invention.

Among them, carbonate apatite, tricalcium phosphate, and octacalcium phosphate exhibit bioabsorbability and therefore also satisfy the demand (7) and as such, constitute an excellent medical honeycomb structure. A medical honeycomb structure comprising bone composition carbonate apatite in composition is an ideal medical honeycomb structure because of its high bone conductivity and adaptation to bone remodeling.

A polymer material comprising at least one bioabsorbable material selected from the group consisting of collagen, chitosan, polyglycolic acid, polylactic acid, poly(lacticco-glycolic acid), and poly(lactide-co-caprolactone) in composition is particularly preferred as composition contained in the medical honeycomb structure of the present invention.

A composite material comprising carbonate apatite and collagen in composition may be particularly preferred because of having both tissue conductivity and flexibility.

### <10 Particularly preferred composition of medical tissue reconstruction material>

The composition of the medical tissue reconstruction material incorporated in the medical tissue reconstruction bag of the present invention is not particularly limited and preferably comprises ceramic or a polymer material excellent in tissue affinity. For use in bone tissue reconstruction surgery, ceramic comprising at least one member selected from the group consisting of carbonate apatite, apatite, tricalcium phosphate, octacalcium phosphate, calcium phosphate, calcium carbonate, calcium sulfate and calcium-containing glass in composition is particularly preferred as composition contained in the medical tissue reconstruction material.

Among them, carbonate apatite, tricalcium phosphate, and octacalcium phosphate exhibit bioabsorbability and therefore also satisfy the demand (7) and as such, constitute an excellent medical tissue reconstruction material. A medical honeycomb structure comprising bone composition carbonate apatite in composition is an ideal medical tissue reconstruction material because of its high bone conductivity and adaptation to bone remodeling.

A polymer material comprising at least one bioabsorbable material selected from the group consisting of collagen, chitosan, polyglycolic acid, polylactic acid, poly(lacticco-glycolic acid), and poly(lactide-co-caprolactone) in composition is particularly preferred as composition contained in the medical tissue reconstruction material.

A composite material comprising carbonate apatite and collagen in composition may be particularly preferred because of having both tissue conductivity and flexibility.

### <11 Method for producing medical honeycomb structure>

The method for producing a medical honeycomb structure according to the present invention is not particularly limited. For example, a general honeycomb structure having an outer peripheral side wall on the entire surface, which is not included in the scope of the present invention, is processed by cutting or the like so that a specific structure of the medical honeycomb structure of the present invention can be imparted thereto to produce the medical honeycomb structure of the present invention. Meanwhile, a method for producing a medical honeycomb structure may be useful, comprising the step of changing the ratio between an extrusion rate from a honeycomb molding die and a rate at which the extruded honeycomb structure is transferred so as to move away from the honeycomb molding die.

The general honeycomb structure preferably has the same size in the through-hole direction. Hence, the extruded honeycomb structure is transferred so as to move away from the honeycomb molding die at the same rate as that of the honeycomb structure extruded from the honeycomb molding die, or at a constant rate ratio using a belt conveyor or the like.

For the medical honeycomb structure of the present invention, it may be preferred that groove side wall 22 should have an undercut in the through-hole direction, as shown in Figure 7a). The honeycomb structure at a stage extruded from the honeycomb molding die is soft and is thinned when pulled in the direction of extrusion. The groove side wall 22 is thinned or thickened by changing the ratio between a rate at which the honeycomb structure is extruded from the honeycomb molding die and a rate at which the extruded honeycomb structure is transferred so as to move away from the honeycomb molding die. As a result, the present medical honeycomb structure comprising groove side wall 22 having an undercut can be produced.

### <12 Production step that renders groove side wall insusceptible to breakage>

Groove side walls 22 are useful for satisfying the demands (1) to (4), while are susceptible to breakage. The groove side walls are susceptible to breakage, particularly, in processing the medical honeycomb structure. The present invention [12] is effective for preventing breakage. This is presumably because a polymer added at least partially to grooves 21 can reduce stress to be applied to groove side walls 22 upon processing such as cutting. The type of the polymer to be added to the grooves is not particularly limited. A wax is preferred because of the ease of addition to the grooves and removal of the polymer after processing. A wax having a lower melting point than that of a wax contained in a raw material is more preferred.

### <13 Specific degreasing step>

In a process of extruding a honeycomb starting material consisting of calcium carbonate and a polymer from a honeycomb mold, processing the produced polymercontaining honeycomb structure into honeycomb structures in the form of granules, and heating and degreasing a layer of the granular honeycomb structure, a plurality of granular honeycomb structure may adhere to each other and fail to produce independent granular honeycomb structures. The present invention [13] is effective for preventing the adhesion.

Such adhesion occurs because the polymer is melted by the heating and degreasing of the granular honeycomb structures and causes the granular honeycomb structures to adhere to each other. Therefore, a powder or granules of inorganic matter can be interposed between the honeycomb structures, thereby suppressing the adhesion between the honeycomb structure and another honeycomb structure. The powder or the granules of inorganic matter are not limited by particle size and may preferably comprise an inorganic matter powder having a volume of 5 × 10⁻¹² m³ or smaller because attachment to the honeycomb structure surface is preferred. On the other hand, in the case of an inorganic matter powder having a small volume, the granular honeycomb structures may adhere to each other through the powder. From this viewpoint, it may be preferred that the inorganic matter powder having a volume of 5 × 10⁻¹² m³ or smaller should be mixed with an inorganic matter powder having a volume of 3 × 10⁻¹¹ m³ or larger.

The powder or the granules of inorganic matter are not limited by composition. Residues of impurities in product composition are not favorable for medical use honeycomb structures. Hence, water-soluble inorganic matter, for example, sodium chloride or potassium chloride, is preferred from the viewpoint that the inorganic matter can be completely removed by washing with water. The solubility of the inorganic matter in water is preferably 5 or more, more preferably 10 or more, further preferably 20 or more.

Hereinafter, the present invention will be described in detail with reference to Examples. However, the scope of the present invention is not limited by these Examples.

The raw material used was a mixture of calcium carbonate and a wax binder. The extrusion molding machine used was Labo Plastomill manufactured by Toyo Seiki Co., Ltd. A honeycomb structure was produced through a honeycomb molding die. Hereinafter, the mixture of calcium carbonate and a wax binder is referred to as a raw material, and the extruded honeycomb structure is referred to as a wax-containing calcium carbonate honeycomb structure. As a binder is a polymer mixture, a binder containing a wax is referred to as a wax binder.

Composition was analyzed using a powder X-ray diffraction apparatus model D8 ADVANCE manufactured by Bruker Japan K.K. under conditions involving an output of 40 kV, 40 mA, and CuKα (λ = 0.15418 nm) as an X-ray source. A sample that seemed to form carbonate apatite was confirmed to be carbonate apatite using a Fourier transform infrared spectrometer model 6200 manufactured by JASCO Corp.

The honeycomb structure was structurally analyzed under a scanning electron microscope model S3400N manufactured by Hitachi High-Tech Corp. or a stereoscopic microscope model MVX10 manufactured by Olympus Corp.

Calcium carbonate was phosphorylated by immersion in a 1 mol/L aqueous Na₂HPO₄ solution of 80°C for 7 days. In general, carbonate apatite containing 3 to 13% by mass of a carbonate group in the apatite structure is produced by the phosphorylation of calcium carbonate. All the carbonate group contents of carbonate apatite produced in the present Examples fell within the range of 8 to 13% by mass. Therefore, these individual carbonate group contents are not particularly described.

A heating and degreasing step, albeit slightly decreasing a size, resulted in an analogous form and was found to cause no essential change. Phosphorylation maintained macromorphology, though changing composition.

### Example 1

A quadrangular prismatic medical wax-containing calcium carbonate honeycomb structure lacking the entire surface of an outer peripheral wall and comprising grooves on the entire surface was produced by extrusion molding using medical honeycomb structure molding die 50 of the present invention that satisfies conditions of the present invention [5] and comprises all outermost peripheral pins 52 contacting the inner face of mask 51. The structure has at least four surfaces including groove side wall distant surfaces 27. The structure was heated and debindered to produce a medical calcium carbonate honeycomb structure. The medical calcium carbonate honeycomb structure was phosphorylated to produce a medical carbonate apatite honeycomb structure.

The medical carbonate apatite honeycomb structure shown in Figure 12a) has portions lacking groove side wall distant surfaces 27 and groove side wall side surfaces 28 because the raw material did not completely enter inter-pin grooves 54 formed by outermost peripheral pins shown in a schematic view of Figure 10. Specifically, this medical honeycomb structure comprises specific groove side walls 22 having an undercut with respect to both directions of through-holes.

Many sites having an undercut index of 1.1 to 4 corresponding to specific groove side walls 22 with groove side wall distant surfaces 27 having an undercut with respect to the through-hole direction were confirmed. Also, many sites having an undercut index of 1.2 to 5 corresponding to specific groove side walls 22 with groove side wall side surfaces 28 having an undercut with respect to the through-hole direction were confirmed.

The medical honeycomb structure shown in Figure 12b) was produced by producing a medical calcium carbonate honeycomb structure, and then grinding a portion of groove side wall distant surfaces 27 using a file, followed by phosphorylation. The medical carbonate apatite honeycomb structure is a medical honeycomb structure comprising specific groove side walls 22 with groove side wall distant surfaces 27 having an undercut with respect to the through-hole direction, and many concave-convex sites having an undercut index of 1.1 to 1.6 existed in the groove side walls 22. The concave-convex sites were disposed at a ratio of at least one or more per groove side wall 22.

The medical carbonate apatite honeycomb structure shown in Figure 12c) was obtained by controlling the cooling rate of a medical wax-containing calcium carbonate honeycomb structure produced by extrusion molding, and increasing the widths of distant sections of groove side walls 22 through self-weight. The medical carbonate apatite honeycomb structure is a medical honeycomb structure comprising specific groove side walls 22 having an undercut with respect to a mesial direction perpendicular to groove bottom wall distant surfaces 26, and many sites having an undercut index of 1.07 to 1.1 were confirmed in the groove side walls 22.

### Example 2

A quadrangular prismatic medical wax-containing calcium carbonate honeycomb structure lacking the entire surface of an outer peripheral wall and comprising grooves on the entire surface was produced by extrusion molding using a honeycomb molding die having no raw material supply hole outside the outermost peripheral pins. The structure has at least four surfaces including groove side wall distant surfaces 27. The structure was heated and debindered to produce a medical calcium carbonate honeycomb structure. The medical calcium carbonate honeycomb structure was phosphorylated to produce the medical carbonate apatite honeycomb structure shown in Figure 12d).

The groove side walls of the medical carbonate apatite honeycomb structure has a wavy structure where the groove side walls are curved a plurality of times along the through-hole direction. This medical carbonate apatite honeycomb structure comprises specific groove side walls 22 that had both groove side wall distant surfaces 27 and groove side wall side surfaces 28 having an undercut with respect to both directions of through-holes, and also has an undercut with respect to a distant direction perpendicular to groove bottom wall distant surfaces 26. Many sites having an undercut index of 1.1 to 1.3 corresponding to specific groove side walls 22 with groove side wall distant surfaces 27 having an undercut with respect to the through-hole direction were confirmed. Many sites having an undercut index of 1.1 to 5 corresponding to specific groove side walls 22 with groove side wall side surfaces 28 having an undercut with respect to the through-hole direction were confirmed. Many sites having an undercut index of 3 to 10 corresponding to specific groove side walls 22 having an undercut with respect to a distant direction perpendicular to groove bottom wall distant surfaces 26 were confirmed.

### Example 3

By using medical honeycomb structure molding die 50 of the present invention that satisfies conditions of the present invention [5] and in which all outermost peripheral pins 52 contact the inner face of mask 51, extrusion molding was performed, and the ratio between an extrusion rate from the honeycomb molding die and the rate of a belt conveyor at which the extruded honeycomb structure was transferred so as to move away from the honeycomb molding die was changed. The structure has four surfaces including groove side wall distant surfaces 27. The structure was heated and debindered to produce a medical calcium carbonate honeycomb structure. The medical calcium carbonate honeycomb structure was phosphorylated to produce a medical carbonate apatite honeycomb structure. The medical honeycomb structure itself had sites differing in width and height, and groove side walls 22 at these sites also differed in width and height. Specifically, the medical honeycomb structure is a medical honeycomb structure comprising specific groove side walls 22 with groove side wall distant surfaces 27 and groove side wall side surfaces 28 having an undercut with respect to the through-hole direction. Many sites having an undercut index of 1.1 to 4 corresponding to specific groove side walls 22 with groove side wall distant surfaces 27 having an undercut with respect to the through-hole direction were confirmed. Also, many sites having an undercut index of 1.2 to 1.5 corresponding to specific groove side walls 22 with groove side wall side surfaces 28 having an undercut with respect to the through-hole direction were confirmed.

### Example 4

A quadrangular prismatic medical wax-containing calcium carbonate honeycomb structure lacking the entire surface of an outer peripheral wall and comprising grooves on the entire surface, wherein the value obtained by dividing the width of groove side wall 22 by the width of groove 21 shown in Figure 3 is less than 1 was produced by extrusion molding, by using medical honeycomb structure molding die 50 that satisfies conditions of the present invention [5] and comprises all outermost peripheral pins 52 contacting the inner face of mask 51, wherein square pins 52 except for the outermost peripheral pins shown in Figure 10 has a side of 0.5 mm; square pins 52 disposed at the top of the outermost periphery has a side of 0.3 mm; rectangular pins except for the top of the outermost periphery has a length of 0.5 mm and a width of 0.3 mm; and the interval between the pins is 0.2 mm, and medical honeycomb structure molding die 50 wherein square pins 52 has a side of 0.3 mm and the interval between the pins is 0.1 mm. The structure has at least four surfaces including groove side wall distant surfaces 27.

The medical honeycomb structure was cut into a substantial cube having an apparent volume of substantially 8 × 10⁻⁹ m³ and a honeycomb aspect ratio of substantially 0.7 using a dental disc. Some groove side walls 22 were broken by this cutting step so as to come off from groove bottom walls 23. A wax having a lower melting point than that of the wax used in the raw material was applied in a melted state to the entire groove surface of the medical wax-containing calcium carbonate honeycomb structure and cured, followed by cutting. This suppressed the breakage of the groove side walls 22.

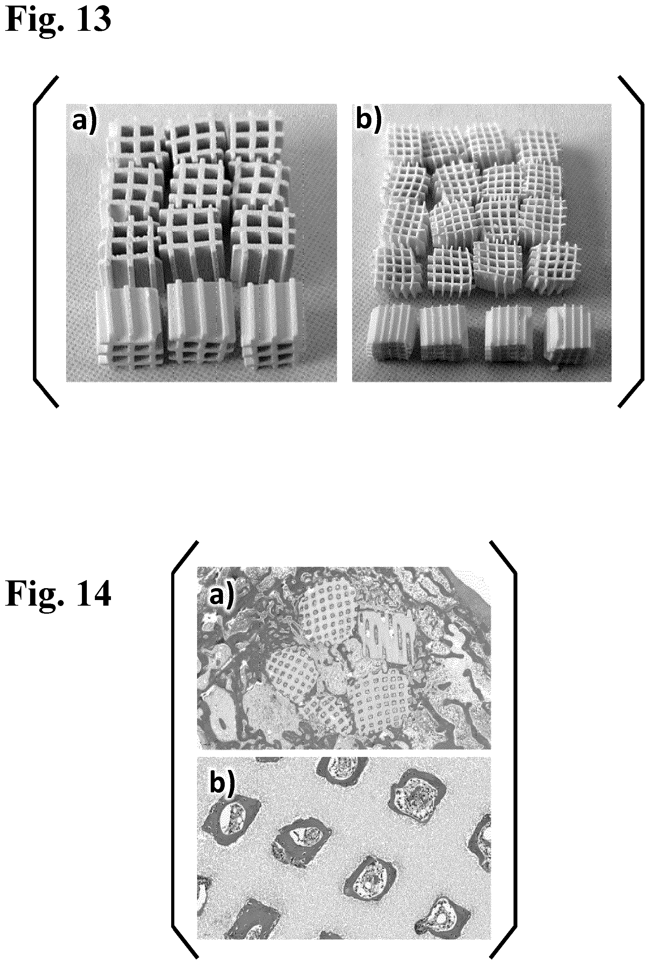

The medical wax-containing calcium carbonate honeycomb structure in the form of granules produced by the application and curing of a wax having a lower melting point in a melted state, followed by cutting, and the medical wax-containing calcium carbonate honeycomb structure in the form of granules produced without the application of a wax having a lower melting point were heated and debindered to both produce medical calcium carbonate honeycomb structure in the form of granules, some of which however adhered to each other. The medical wax-containing calcium carbonate honeycomb structure in the form of granules were mixed with a mixed powder of sodium chloride manufactured by Tomita Pharmaceutical Co., Ltd. (Tomita Salt K30) which had a particle size of substantially 35 µm at a cumulative value of 50% in a particle size distribution determined by the laser diffraction/scattering method, and had a volume of substantially 4.3 × 10⁻¹⁴ m³, and sodium chloride manufactured by FUJIFILM Wako Pure Chemical Corp. (Guaranteed Reagent) which had a volume of substantially 2 × 10⁻¹⁰ m³ so that the sodium chloride powder was interposed between the honeycomb structure in the form of granules, followed by heating and degreasing, and washing with water. This produced medical calcium carbonate honeycomb structure in the form of granules without adhering to each other. The medical calcium carbonate honeycomb structure was phosphorylated to produce the medical carbonate apatite honeycomb structure in the form of granules shown in Figure 13.

Some granules shown in Figures 13a) and 13b) comprise groove side walls having an undercut with respect to a mesial direction perpendicular to groove bottom wall distant surfaces 26. Many sites having an undercut index of 1.3 to 1.5 corresponding to specific groove side walls 22 that were inclined and having an undercut with respect to a mesial direction perpendicular to groove bottom wall distant surfaces 26 were confirmed. The groove side walls of the medical calcium carbonate honeycomb structure shown in Figure 13a) were insusceptible to breakage because this medical honeycomb structure satisfied the requirements (A) and (B) such that the value obtained by dividing the height of the groove side wall by the width of the groove side wall was substantially 1.3 or less and the value obtained by dividing the height of the groove side wall by the width of the groove was substantially 0.7 or less. Furthermore, the medical calcium carbonate honeycomb structure shown in Figures 13a) and 13b) in the form of granules were found to be fixed to each other by the entry of groove side wall 22 of one granule of the medical honeycomb structure into groove 21 of another granule of the medical honeycomb structure because this medical honeycomb structure satisfied the requirement (C) such that the value obtained by dividing the width of the groove side wall by the width of the groove was substantially 0.5 and 0.3, respectively.

The medical honeycomb structure of the present invention shown in Figure 13a) or 13b) was granules and was therefore easy to implant in a tissue defect. This medical honeycomb structure also had an apparent volume of 1 × 10⁻⁷ m³ or smaller and was thus found to have a small distance between the granules and a high packing density in a tissue defect.

Figure 14 shows a hematoxylin-eosin staining image on week 4 when a bone defect of the internal condyle of the rabbit femur was reconstructed with a carbonate apatite medical honeycomb structure produced in the same manner as above using medical honeycomb structure molding die 50 shown in Figure 10 wherein square pins 52 had a side of 0.15 mm and the interval between the pins was 0.15 mm. Despite week 4 after the surgery, bones were conducted to the inside of through-holes 25 and the surface of groove side walls 22 in the carbonate apatite medical honeycomb structure. The material was therefore confirmed to strongly bind to surrounding tissues and to have a large rate of bone tissue reconstruction in a bone defect. It is also confirmed that the conducted bones were oriented in the through-hole direction. Not only bone conduction but vascularization was found in the inside of the through-holes. It is thus found that bones having a Haversian canal structure were formed. Furthermore, osteoblasts and osteoclasts were also confirmed. It is thus found that the carbonate apatite medical honeycomb structure was replaced with bones.

### Comparative Example 1

A wax-containing calcium carbonate honeycomb structure was produced under the same conditions as in Example 1, except that the general honeycomb molding die of Figure 9 that was not included in the scope of the present invention in which outermost peripheral pins 42 were not in contact with the inner face of mask 41 and pin outer periphery 45 was provided was used.

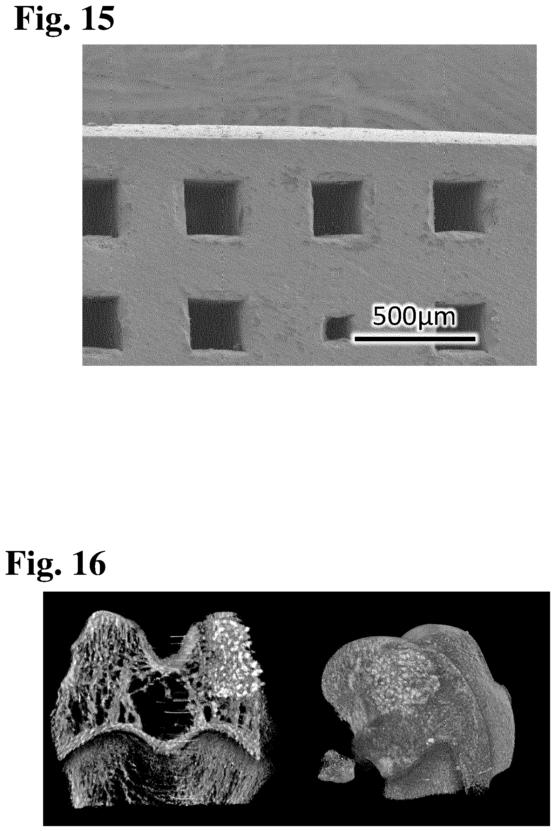

The structure was heated and debindered to produce a calcium carbonate honeycomb structure. The medical calcium carbonate honeycomb structure was phosphorylated to produce a carbonate apatite honeycomb structure. As shown in Figure 15, the honeycomb structure had outer peripheral side wall 13 on the entire outer peripheral side surface. Thus, this general honeycomb structure was not included in the scope of the present invention.

Due to the presence of the outer peripheral side wall on the entire outer peripheral side surface, the outer peripheral side surface had a small contact area with surrounding tissues. This structure had no undercut. Thus, the honeycomb structure had limited mechanical binding force to surrounding tissues, and was also found to have a relatively small rate of tissue defects reconstruction in a tissue defect due to the absence of groove side walls.

### Example 5

Two sheets of Vicryl Mesh (knit-type mesh made of glycolic acid/lactic acid polyester) manufactured by Johnson & Johnson K.K. were sutured to produce a 20 mm wide and 40 mm long mesh bag having one introduction port. Then, four corners of the bag were fused with a sealer, and a hole having a diameter of 1.2 mm through which a screw would pass was opened at the fused sites. The area of one mesh hole 61 of the bag was substantially 1. 1 × 10⁻⁷ m², and the value obtained by dividing the total opening area of the mesh by the area of the mesh bag was substantially 0.42. In order to suit its form to a rectangular tissue defect form, the bag was allowed to incorporate substantially 2 × 10⁻⁶ m³ of carbonate apatite medical honeycomb structure in the form of granules having a volume of substantially 1 × 10⁻⁹ m³ per granule as a medical tissue reconstruction material from the introduction port. As a result, the granules in the bag were movable due to insufficient contact between the granules. For a high level of contact between the granules, substantially 5 × 10⁻⁶ m³ of the medical honeycomb structure was incorporated in the bag. As a result, the central part was enlarged so that the bag became a cylindrical form, which was different from the tissue defect form, though a high level of contact between the granules was attained. Hence, non-stretchable inside string 63 for reduction in bag enlargement which could connect two inside locations of medical tissue reconstruction bag 60 was provided at 15 sites at substantially 5-mm intervals so as to connect the facing surfaces of the bag. 2 × 10⁻⁶ m³ of the medical honeycomb structure was incorporated in the bag, and the introduction port was closed by suture to successfully produce a medical tissue reconstruction bag incorporating a medical tissue reconstruction material in a form relatively adapted to the tissue defect form, wherein the granules were in sufficiently tight contact with each other.

### Example 6

S212ZP manufactured by Collant, Inc., which was a stretchable nylon/polyurethane mesh, was sutured to produce a 3 mm wide and 7 mm long bag having one introduction port. The bag elongated to substantially 11 mm and substantially 25 mm under a load of 10 N in the length direction and the width direction, and had a strain of substantially 3.5 under these conditions. The strain in other directions was also substantially 3.5. The area of one mesh hole 61 of the bag was substantially 1.0 × 10⁻⁶ m², and the value obtained by dividing the total opening area of the mesh by the area of the mesh bag was substantially 0.6. The bag was allowed to incorporate substantially 8.5 × 10⁻⁸ m³ of carbonate apatite granules (Cytrans Granules S Size manufactured by GC Corp.) having a volume of substantially 1.4 × 10⁻¹¹ m³ to 1.1 × 10⁻¹⁰ m³ as a medical tissue reconstruction material from the introduction port. The bag was fused with a sealer while drawn. As a result, the granules were in sufficient contact with each other because of the stretchability of the bag.

A bone defect having a diameter of 6 mm was formed in the internal condyle of the rabbit femur and subjected to a procedure of a bone reconstruction surgery using the mesh bag incorporating the carbonate apatite medical honeycomb structure granules. A µCT image taken after 4 weeks is shown in Figure 16. In the procedure of the bone reconstruction surgery with only the carbonate apatite medical honeycomb structure in the form of granules, a portion of the structure was leaked out. In the procedure of the bone reconstruction surgery using the mesh bag incorporating the structure, it was confirmed that the structure was not leaked out. It was also confirmed that bones were conducted from existing bones to the structure surface through the mesh introduction port.

### Example 7

The mesh bag used in Example 5 comprised mesh holes 61 having an area of substantially 1.2 × 10⁻⁷ m². Therefore, when the bag was allowed to incorporate substantially cubic carbonate apatite medical honeycomb structure in the form of granules having a volume of substantially 1.2 × 10⁻¹⁰ m³ per granule as a medical reconstruction material from the introduction port 62, a portion of the structure was discharged from the mesh holes 61 of the bag to the outside of the bag. The bag was fused with a polyglycolic acid suture thread Opepolix N (6-0) manufactured by Alfresa Pharma Corp. such that the mesh holes 61 of the bag were halved with the suture thread. Since the suture thread occupied a portion of the mesh holes, the area of the mesh holes 61 became substantially 4 × 10⁻⁸ m² and the value obtained by dividing the total opening area of the mesh by the area of the mesh bag became substantially 0.3. The medical tissue reconstruction bag was allowed to incorporate carbonate apatite medical honeycomb structure in the form of granules having a volume of substantially 1.2 × 10⁻¹⁰ m³ per granule, and the introduction port 62 was sutured. As a result, the honeycomb structure was not discharged to the outside of the bag. Since the carbonate apatite medical honeycomb structure in the form of granules having a small volume were incorporated in the medical tissue reconstruction bag, the interval between the carbonate apatite medical honeycomb structure in the form of granules was small.

### Explanation of Reference Signs

10: general honeycomb structure not included in the present invention
11: through-holes
12: partition wall
13: outer peripheral side wall
20: medical honeycomb structure of the present invention
21: groove
22: groove side wall
23: groove bottom wall
24: partition wall
25: through-holes
26: groove bottom wall distant surface
27: groove side wall distant surface
28: groove side wall side surface
29: base point
30: another point
31: counter base point
32: another counter point
33: reference plane
40: general honeycomb molding die not included in the present invention (one example)
41: mask
42: pin
43: material supply hole
44: inter-pin groove
45: pin outer periphery
50: medical honeycomb structure molding die of the present invention (one example)
51: mask
52: pin
53: material supply hole
54: inter-pin groove
60: medical tissue reconstruction bag of the present invention (one example)
61: mesh hole
62: introduction port
63: inside string for reduction in bag enlargement (one example)
64: surface string for reduction in bag enlargement (one example)

## Claims

1. A medical honeycomb structure lacking at least a portion of an outer peripheral side wall of a honeycomb structure comprising a plurality of through-holes extending in one direction, wherein sites lacking the outer peripheral side wall comprise a plurality of grooves, and comprise a plurality of planes including distant surfaces of groove side walls flanked by the grooves, with the proviso that a medical honeycomb structure wherein the sites lacking the outer peripheral side wall comprising grooves and through-hole inlets adjacent to the grooves is excluded.

2. The medical honeycomb structure according to claim 1, wherein the medical honeycomb structure comprises a groove side wall having an undercut in a through-hole direction and/or in a direction perpendicular to a distant surface of a groove bottom wall.

3. The medical honeycomb structure according to claim 1 or 2, wherein the medical honeycomb structure comprises a groove side wall that satisfies at least one of the following (A) to (C):
(A) a value obtained by dividing a height of the groove side wall by a width of the groove side wall is 3 or less;
(B) a value obtained by dividing the height of the groove side wall by the width of the groove is 0.8 or less; and
(C) a value obtained by dividing the width of the groove side wall by the width of the groove is less than 1.

4. The medical honeycomb structure according to any one of claims 1 to 3, wherein the medical honeycomb structure has a honeycomb aspect ratio obtained by dividing a maximum length of the through-hole or the groove by a major axis of a plane perpendicular to the through-hole of 3 or less, and an apparent volume of 1 × 10⁻⁷ m³ or smaller.

5. A medical honeycomb structure molding die for use in production of a medical honeycomb structure lacking at least a portion of an outer peripheral side wall of a honeycomb structure comprising a plurality of through-holes extending in one direction, wherein sites lacking the outer peripheral side wall comprise a plurality of grooves, and comprise a plurality of planes including distant surfaces of groove side walls flanked by the grooves, wherein at least one outermost peripheral pin and a mask inner face are substantially in contact and/or are integrated with each other.

6. A medical tissue reconstruction bag comprising a mesh section in at least one portion, wherein a mesh opening area is 2 × 10⁻⁹ m² or larger, and a value obtained by dividing a total opening area of the mesh by an area of the mesh section is 0.2 or more,
comprising an introduction port for incorporation of a medical tissue reconstruction material, or incorporating a medical tissue reconstruction material without an introduction port, and
satisfying at least one requirement selected from the group of (D) to (F):
(D) having a mesh opening area of 2 × 10⁻⁵ m² or smaller, and comprising a structure that reduces enlargement of the bag associated with the incorporation of a material;
(E) having a mesh opening area of 2 × 10⁻⁵ m² or smaller, and exhibiting a strain of 0.2 or more under a load of 10 N in an arbitrary direction; and
(F) having a mesh opening area of 1.0 × 10⁻⁷ m² or smaller.

7. A medical tissue reconstruction bag comprising a mesh section in at least one portion, wherein a mesh opening area is 2 × 10⁻⁹ m² or larger and 2 × 10⁻⁵ m² or smaller, and a value obtained by dividing a total opening area of the mesh by an area of the mesh section is 0.2 or more, and incorporating a carbonate apatite medical tissue reconstruction material.

8. The medical tissue reconstruction bag according to claim 6 or 7, wherein the medical tissue reconstruction bag comprises a hole and/or a thread for fixing the medical tissue reconstruction bag to a tissue.

9. The medical honeycomb structure according to any one of claims 1 to 4, wherein the medical honeycomb structure comprises at least one selected from the group consisting of carbonate apatite, apatite, tricalcium phosphate, octacalcium phosphate, calcium phosphate, calcium carbonate, calcium sulfate, calcium-containing glass, collagen, chitosan, polyglycolic acid, polylactic acid, poly(lactic-co-glycolic acid), and poly(lactideco-caprolactone) in composition.

10. The medical tissue reconstruction bag according to any one of claims 6 to 8, wherein the medical tissue reconstruction material comprises at least one selected from the group consisting of carbonate apatite, apatite, tricalcium phosphate, octacalcium phosphate, calcium phosphate, calcium carbonate, calcium sulfate, calcium-containing glass, collagen, chitosan, polyglycolic acid, polylactic acid, poly(lactic-co-glycolic acid), and poly(lactide-co-caprolactone) in composition.

11. A method for producing a medical honeycomb structure, comprising a step of producing a medical honeycomb structure lacking at least a portion of an outer peripheral side wall of a honeycomb structure comprising a plurality of through-holes extending in one direction, wherein sites lacking the outer peripheral side wall comprise a plurality of grooves,
the production step comprising a step of changing a ratio of an extrusion rate from a honeycomb molding die to a rate at which an extruded honeycomb structure is transferred so as to move away from the honeycomb molding die.

12. A method for producing a medical honeycomb structure, comprising a step of producing a medical honeycomb structure lacking at least a portion of an outer peripheral side wall of a honeycomb structure comprising a plurality of through-holes extending in one direction, wherein sites lacking the outer peripheral side wall comprise a plurality of grooves,
the production step comprising adding a polymer at least to a portion of the grooves at the sites lacking the outer peripheral side wall, and then processing the medical honeycomb structure, followed by removal of the polymer.

13. A method for producing a medical honeycomb structure, comprising a step of heating and degreasing a layer of a plurality of honeycomb structures containing a polymer, wherein a powder and/or granules of inorganic matter are interposed between the honeycomb structures.
